# EUROPÄISCHE PATENTANMELDUNG

(11) **EP 4 434 608 A1**
(43) Veröffentlichungstag der Anmeldung: **25.09.2024**
(21) Anmeldenummer: 23164019.4
(22) Anmeldetag: 24.03.2023
(51) Int. Cl.: B01D 53/04, A61M 16/10, C01B 13/02, A61M 16/16

(54) **TRAGBARER SAUERSTOFFKONZENTRATOR MIT VORGESCHALTETER FILTEREINHEIT**

(71) Anmelder: Axnar Med Tech GmbH, 22453 Hamburg (DE)
(72) Erfinder: Jochen, Hölscher, Hamburg (DE)
(74) Vertreter: Patentanwälte Olbricht Buchhold Keulertz

(57) **Zusammenfassung**

Gegenstand der Erfindung ist eine tragbare Sauerstoffversorgungsvorrichtung umfassend einen Sauerstoffkonzentrator, mindestens einen Lufteinlass durch den Umgebungsluft in die Sauerstoffversorgungsvorrichtung transportiert wird, einen Sauerstoff-Auslass, durch den angereicherter Sauerstoff aus der Sauerstoffversorgungsvorrichtung transportiert wird, einen Abluftauslass, durch den Abluft aus der Sauerstoffversorgungsvorrichtung transportiert wird, ein Gasleitungssystem, das mit dem mindestens einen Lufteinlass, dem Auslass und dem Sauerstoffkonzentrator fluidisch verbunden ist, eine Stromquelle, mindestens eine Pumpe und ein Gehäuse, wobei alle Komponenten der Sauerstoffversorgungsvorrichtung im oder am Gehäuse angeordnet sind und wobei der Lufteinlass einen Ansaugstutzen aufweist, durch den die Umgebungsluft in die Sauerstoffversorgungsvorrichtung gesogen wird. Die Sauerstoffversorgungsvorrichtung weist eine Filtereinheit auf. Gegenstand der Erfindung ist weiterhin ein Sauerstoffversorgungssystem aufweisend eine erfindungsgemäße Sauerstoffversorgungsvorrichtung und eine fahrzeugtaugliche Gerätehalterung mit Sicherung. Die Filtereinheit schützt die Sauerstoffversorgungsvorrichtung vor einer Kontamination oder Beschädigung durch nukleare, biologische oder chemische Verunreinigungen oder Stoffe oder große Partikel.

## Beschreibung

Gegenstand der Erfindung ist eine tragbare Sauerstoffversorgungsvorrichtung zur Versorgung eines Patienten gemäß dem Oberbegriff von Patentanspruch 1 sowie ein Beatmungssystem gemäß Patentanspruch 11.

Zur Sauerstoffversorgung von Patienten, insbesondere bei der Notfallversorgung oder der häuslichen Versorgung von chronisch kranken Patienten wird Sauerstoff aus Flaschen oder aus einem Sauerstofferzeuger, wie z.B. einem Sauerstoffkonzentrator, eingesetzt. Ein Sauerstoffkonzentrator saugt die Luft aus seiner unmittelbaren Umgebung über eine Pumpe an. Die angesaugte Luft durchströmt dabei mehrere Filter, um Verschmutzungen oder Keime zu beseitigen. Die gereinigte Luft wird anschließend verdichtet. Der in der Luft enthaltene Stickstoff wird durch einen Adsorber, das sogenannte Molekularsieb, adsorbiert und von der restlichen Luft abtrennt. Somit erhöht sich der Sauerstoff in der abgegebenen Luft von > 21 % auf bis zu 96%.

In der abschließenden Phase wird der gefilterte Stickstoff im Sauerstoffkonzentrator von den Molekularsieben entfernt und der konzentrierte Sauerstoff an den Patienten abgegeben. Nachdem die Adsorber regeneriert wurden, beginnt der Prozess erneut.

Um die kontinuierliche und stetige Zufuhr von konzentriertem Sauerstoff zu gewährleisten, arbeiten Sauerstoffkonzentratoren mit zwei, der oben beschriebenen Systeme parallel, sodass sich immer ein System in der Phase Ansaugen, Konzentration und Regeneration befindet, und dadurch die stetige Zufuhr von konzentriertem Sauerstoff zum Patienten gewährleistet ist.

Herkömmliche Sauerstoffkonzentratoren werden meist im klinischen oder häuslichen Bereich und somit in einer relativ sauberen Umgebung eingesetzt. Sie verfügen deshalb nur über Filter, die vor groben Verunreinigungen schützen, wie HEPA Filter. Ein HEPA-Filter, Abkürzung für den englischen Begriff "High Efficiency Particulate Air" ist ein mechanischer Filter, der Schwebstoffe aus der Luft filtert.

Während des Einsatzes in der prähospitalen Notfallmedizin, kann es dazu kommen, dass die Einsatzstelle mit atomaren, biologischen oder chemischen Stoffen verunreinigt ist. Dadurch werden mögliche Stoffe in das Gerät und im Anschluss an den Patienten direkt weitergeleitet. Dieses hat zur Folge das Gerät nach einem Einsatz nicht weiterverwendet werden und der Patient nicht geschützt wird.

Heutige Sauerstoffkonzentratoren sind nur für den Einsatz im "sicheren Bereichen" wie im häuslichen und Krankenhausumfeld ausgelegt. Dadurch werden nur HEPA-Filter verwendet, die keinen Schutz vor Kontaminationen des Patienten oder des Geräts bieten, sondern vor Beschädigung der Pumpe schützen sollen.

WO 03/015899 A1 beschreibt einen Sauerstoffkonzentrator auf Basis einen VPSA-System mit einem Filter. Der Filter ist vor dem Kompressor angeordnet, damit Partikel vor dem Kompressor herausgefiltert werden und diesen vor mechanischer Beschädigung schützen.

Aufgabe der vorliegenden Erfindung ist es daher, eine Sauerstoffversorgungsvorrichtung bereitzustellen, die die vorstehenden Nachteile überwindet und einen sicheren Einsatz auch in der prähospitalen Notfallmedizin erlaubt.

Hauptmerkmale der Erfindung sind im kennzeichnenden Teil von Anspruch 1 angegeben. Ausgestaltungen sind Gegenstand der Ansprüche 2 bis 10.

Weitere Ausführungsformen sind Gegenstand der Unteransprüche oder nachfolgend beschrieben.

Die erfindungsgemäße tragbare oder stationäre Sauerstoffversorgungsvorrichtung zur Versorgung eines Patienten umfasst
- einen Sauerstoffkonzentrator,
- mindestens einen Lufteinlass, durch den Umgebungsluft in die Sauerstoffversorgungsvorrichtung transportiert wird,
- mindestens einen Sauerstoff-Auslass, durch den angereicherter Sauerstoff aus der Sauerstoffversorgungsvorrichtung transportiert wird,
- mindestens einen Abluftauslass, durch den Abluft aus der Sauerstoffversorgungsvorrichtung transportiert wird,
- ein Gasleitungssystem, das mit dem mindestens einen Lufteinlass, dem Sauerstoff-Auslass, dem Abluftauslass und dem Sauerstoffkonzentrator fluidisch verbunden ist,
- eine Stromquelle,
- mindestens eine Pumpe,
- ein Gehäuse, wobei alle Komponenten der Sauerstoffversorgungsvorrichtung im Gehäuse oder am Gehäuse angeordnet sind.

Der Lufteinlass weist einen Ansaugstutzen auf, durch den die Umgebungsluft in die Sauerstoffversorgungsvorrichtung gesogen wird. Die Sauerstoffversorgungsvorrichtung weist eine Filtereinheit auf, so dass Umgebungsluft erst durch die Filtereinheit strömt und vor Eintritt in die Sauerstoffversorgungsvorrichtung gefiltert wird. Die Filtereinheit ist vor dem Lufteinlass befestigt. Entweder ist sie direkt am Ansaugstutzen selbst befestigt oder mit einem Adapter am Ansaugstutzen befestigt. Die Filtereinheit ist gasdicht mit dem Lufteinlass verbunden. Die Filtereinheit ist ausgewählt aus NBC-Filter, Kombinationsfilter und Partikelfilter.

Bevorzugt ist die Sauerstoffversorgungsvorrichtung ein mobiles oder teilstationäres Gerät, das als Stromquelle mindestens eine Batterieeinheit oder mindestens einen Akkumulator aufweist und ein stoßfestes Gehäuse hat. Unter teilstationär wird beispielsweise ein größeres Gerät in einer Transportbox oder einem Transportcontainer verstanden, dass mit Hilfsmitteln zum Einsatzort transportiert werden kann. Die Sauerstoffversorgungsvorrichtung kann alternativ auch mit herkömmlichen Netzstrom betrieben werden, z.B. über einen Generator.

Die Filtereinheit ist bevorzugt als Filtereinheit für eine Atemschutzmaske, auch NBC-Schutzmaske genannt, ausgebildet. Insbesondere ist die Filtereinheit eine NBC-Filtereinheit für eine Atemschutzmaske, eine Partikelfiltereinheit für einen Atemschutzmaske oder eine Kombinationsfiltereinheit für einen Atemschutzmaske. Die Filtereinheit kann zusätzlich einen HEPA-Filter aufweisen.

Eine NBC- Filtereinheit, auch als ABC-Filtereinheit bezeichnet, schützt vor chemischen Kampfstoffen, giftigen Industriechemikalien, Mikroorganismen und radioaktiven Partikeln. Die Kennzeichnung NBC steht dabei für nuklear, biologisch und chemisch. Der europäische Standard für diese Filter ist die EN 14386 für Industriefilter.

In einer Ausführungsform hat der Ansaugstutzen einen Gewindeanschluss, der auf der der Umgebungsluft zugewandten Seite des Lufteinlasses angeordnet ist. Bevorzugt ist der Gewindeanschluss ein Gewindeanschluss Rd40, d.h. ein Rundgewindeanschluss mit 40 mm x 1/7 Zoll nach Norm EN 148 Teil 1).

Durch die Auslegung des Ansaugstutzens nach dem Standard Gewindeanschluss Rd40 nach EN 148 Teil 1) bzw. NATO Standard STAGNAG 4155 und mit einem Innendurchmesser von 22 mm können handelsübliche NBC-Filter und handelsübliche medizinische Filter für Beatmungsfilter evtl. ergänzt durch zusätzliche Adapter an der Sauerstoffversorgungsvorrichtung angebracht werden, dadurch ist es möglich den Patienten und das Gerät zu schützen. Die Verwendung von Standardfiltern für Atemschutzmasken stellt zudem auch in schwierigen Einsatzgebieten, wie Kriegs- oder Krisengebieten oder bei Notfalleinsätzen, eine gute Verfügbarkeit der Filtereinheiten sicher, da bereits vorhandene Filtereinheiten von Atemschutzmasken übernommen werden können.

Im Ansaugstutzen oder Lufteinlass kann ein Erkennungsmechanismus angeordnet sein, wobei der Erkennungsmechanismus erkennt, ob überhaupt eine Filtereinheit eingesetzt ist und ggf. zusätzlich erkennt, welcher Filtertyp eingesetzt ist. Der Erkennungsmechanismus kann eine oder mehrere Erkennungseinheiten aufweisen.

Der Erkennungsmechanismus kann als Erkennungseinheit einen mechanischen Druckschalter im Gewindeverlauf des Ansaugstutzens aufweisen.

In einer Ausführungsform weist die Sauerstoffversorgungsvorrichtung eine Steuereinheit auf. Der Erkennungsmechanismus ist mit der Steuereinheit verbunden. Die Steuereinheit steuert die Leistung des Sauerstoffkonzentrator. Ggf. ist die Steuereinheit mit einer Informationseinheit verbunden ist, die eine Information zur Anwesenheit einer Filtereinheit und/oder der veränderten Leistung an den Anwender gibt z.B. über ein Display oder eine andere Anzeigevorrichtung.

Der Sauerstoffkonzentrator kann ein Druckwechseladsorptionssystem (PSA) oder ein Vakuum-Druckwechseladsorptionssystem (VPSA) sein und eine oder mehrere Adsorbersäulen aufweisen. Die Adsorber sind bevorzugt Zeolithe. Dieses ist ein spezielles Material zur Adsorption von Stickstoff, wodurch ein sauerstoffreiches Gas gewonnen wird.

Die Sauerstoffversorgungsvorrichtung kann einen oder mehrere Sensoren im Gehäuse aufweisen, die bevorzugt mit dem Gasleitungssystem verbunden sind. Die Sensoren können z.B. Drucksensoren, Temperatursensoren, Gassensoren zur Bestimmung einer Gaskonzentration, Durchflusssensoren, Sensoren zur Erkennung der Stromversorgung oder Feuchtigkeitssensoren sein.

Die Sauerstoffversorgungsvorrichtung kann weitere Bauteile, wie z.B. Schalldämpfer Luftbefeuchter oder Anzeigeeinheiten, wie Displays, aufweisen.

Gegenstand der Erfindung ist weiterhin ein Sauerstoffversorgungssystem aufweisend eine erfindungsgemäße Sauerstoffversorgungsvorrichtung und eine fahrzeugtaugliche Gerätehalterung mit Sicherung. Die Fahrzeughalterung ist bevorzugt eine Gerätehalterung für ein Landfahrzeug, ein Luftfahrzeug oder ein Wasserfahrzeug, bevorzugt für einen Rettungswagen, einen Rettungshubschrauber oder ein Krankentransportflugzeug.

Heutige Sauerstoffkonzentratoren sind nur für den Einsatz in "sicheren Bereichen" wie im häuslichen und Krankenhausumfeld ausgelegt. Die verwendeten HEPA-Filter bieten keinen Schutz vor Kontaminationen.

Die Filtereinheit schützt die erfindungsgemäße Sauerstoffversorgungsvorrichtung vor einer Kontamination oder Beschädigung durch nukleare, biologische oder chemische Verunreinigungen oder Stoffe oder große Partikel.

Die erfindungsgemäße Sauerstoffversorgungsvorrichtung ist besonders für den Einsatz mit enormer Belastung von Sand, wie beim Einsatz im militärischen Kontext in der Wüste oder trockene Gegenden oder bei offenen Türen im Hubschrauber geeignet. Im Hubschrauber kommt es während Start und Landung zum "Downwash". Bei diesen Einsätzen kann es zu massiven Verwirbelungen von Sand kommen. Dieser Sand wird bei herkömmlichen Geräten dann im Ansaugschutz im Sauerstoffkonzentrator angesaugt und kann innere Bauteile zerstören. Bei der erfindungsgemäßen Sauerstoffversorgungsvorrichtung wird der Sand in der Filtereinheit abgefangen und die inneren Bauteile sind so geschützt. Nach dem Einsatz kann die verschmutzte Filtereinheit ausgetauscht werden und das Gerät ist wieder einsatzbereit.

Auch bei Einsatz in infektiöser Umgebung, wie z.B. im Krankenhaus ist die Sauerstoffversorgungsvorrichtung gegen Kontamination geschützt. Herkömmlich wird die Raumlauft in das Gerät eingesaugt, wobei pathogene Keime nicht durch den Standard HEPA Filter aufgehalten werden, somit kann es zu Cross-Kontaminationen zu anderen Patienten kommen. Bei der erfindungsgemäßen Sauerstoffversorgungsvorrichtung werden die Mikroorganismen in der Filtereinheit abgefangen, so dass andere Patienten und Personal vor einer Kontamination geschützt sind. Auch hier kann nach dem Einsatz die kontaminierte Filtereinheit ausgetauscht werden und das Gerät ist wieder einsatzbereit.

Durch den Einsatz von vorgelagerten Filtereinheiten kommt es zu einem Anstieg des Widerstandes am Lufteinlass des Sauerstoffkonzentrators. Um den Druckanstieg zu kompensieren, wird die Leistung der Vakuumpumpe und der Druckpumpe angepasst. Zusätzlich kann der Benutzer über eine Informationseinheit über die geänderten Leistungsparameter, wie z.B. verkürzte Batterielaufzeit, reduzierte Sauerkonzentration oder reduzierte Durchflussleistung informiert werden. Durch die Erkennung der Filtereinheit über den Erkennungsmechanismus im Schraubgewinde kann die Regelung mit einer Steuereinheit selbstständig gestartet und beendet und eine Filtertyperkennung durchgeführt werden, ohne dass der Benutzer eingreifen muss.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1: eine schematische Zeichnung einer erfindungsgemäße Sauerstoffversorgungsvorrichtung,
- Fig. 2: eine Draufsicht auf den Lufteinlass ohne montierter Filtereinheit und
- Fig.: ein Fließbild für den Betrieb einer erfindungsgemäßen Sauerstoffversorgungseinrichtung.

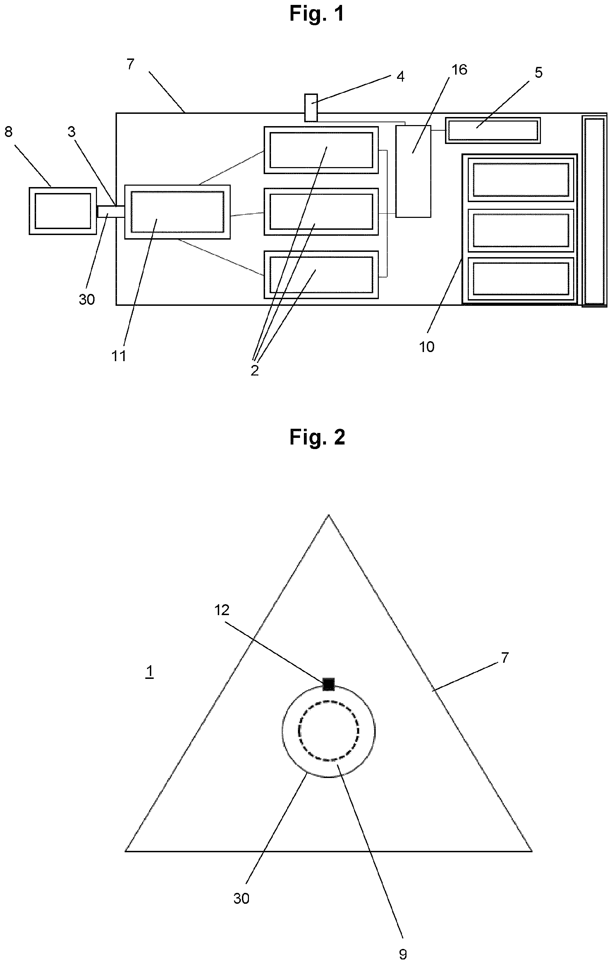

Figur 1 zeigt einen schematischen Aufbau einer erfindungsgemäßen Sauerstoffversorgungsvorrichtung. In einem Gehäuse 7 sind ein Sauerstoffkonzentrator 2, eine Stromquelle 10 und zwei Pumpen 11 und 16 angeordnet. Am Gehäuse befindet sich ein Lufteinlass 3, der durch die Gehäusewand hindurchführt. Der Lufteinlass weist einen Ansaugstutzen 30 an der Außenseite des Gehäuses auf. Vor den Ansaugstutzen ist eine Filtereinheit 8 angeordnet, die mit dem Ansaugstutzen 30 gasdicht verbunden ist. Der Abluftauslass 5 führt ebenfalls durch das Gehäuse 7 und ermöglicht es Abluft aus der Sauerstoffversorgungsvorrichtung zu transportieren. Der Sauerstoffauslass 4 ist mit einem Schlauch verbunden, der zum Patienten führt.

In Figur 2 ist eine Draufsicht auf die Seite der erfindungsgemäßen Sauerstoffversorgungsvorrichtung 1 mit Lufteinlass 3 ohne montierte Filtereinrichtung zu erkennen. Der Lufteinlass führt von der Außenseite durch das Gehäuse 7 in das Innere der Sauerstoffversorgungseinrichtung. Der Lufteinlass 3 ist an der Außenseite des Gehäuses 7 als Ansaugstutzen 30 ausgebildet. Der Ansaugstutzen 30 weist an der Innenseite ein Schraubgewinde 9 auf. Im Schraubgewinde 9 ist ein Schalter 12 angeordnet. Der Schalter 12 dient als Erkennungsmechanismus für eine Filtereinheit (nicht dargestellt).

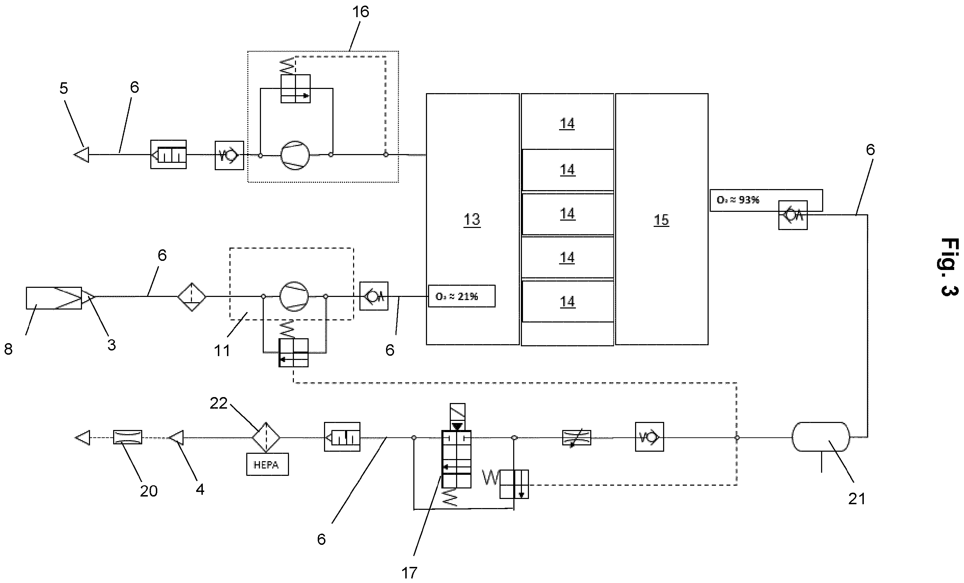

Figur 3 zeigt ein Fließbild für eine Betriebsvariante einer erfindungsgemäßen Sauerstoffversorgungsvorrichtung 1 mit VPSA-System. Umgebungsluft wird durch eine Filtereinheit 8 über den Lufteinlass 3 in das Gasleitungssystem 6 zu einer ersten Druckpumpe 11 gesaugt. Die Druckpumpe 11 transportiert die Umgebungsluft durch das Gasleitungssystem 6 zu einem ersten Verteiler 13, der mehrere Ventile aufweist. Vom Verteiler 13 wird die komprimierte Umgebungsluft zu den Adsorbereinheiten 14 geleitet, so dass die Adsorbereinheiten von der Luft durchströmt werden. In der Adsorbereinheit 14 wird Stickstoff und andere Bestandteile aus der Umgebungsluft adsorbiert, so dass ein Gas mit erhöhtem konzentriertem Sauerstoffanteil entsteht.

Das Gas mit konzentriertem Sauerstoffanteil wird aus der Adsorbereinheit 14 über einen zweiten Verteiler 15, der mehrere Ventile aufweist, und über ein Zwei-Wege-Ventil 17 zum Sauerstoffauslass 4 und von dort über einen Schlauch 20 zum Patienten geführt. Nach einer Weile ist die Adsorbereinheit weitestgehend gesättigt. Zu diesem Zeitpunkt werden die Ventile in dem ersten Verteiler 13 und im zweiten Verteiler 15 so umgeschaltet, dass die Gasleitung zum Sauerstoffauslass 4 geschlossen wird und die Verbindung zum Abluftauslass 5 für die Abluft geöffnet wird. Das adsorbierte Gas wird wieder desorbiert. Durch wechselseitig beladene und entladene Adsorber ist ein kontinuierlicher Betrieb sichergestellt. Bei der Ausführung als VPSA-System weist die Sauerstofferzeugungsvorrichtung in fluidischer Verbindung mit dem ersten Verteiler 13 eine Vakuumpumpe 16 auf. Die Abluft wird aus der Adsorbereinheit 14 über den ersten Verteiler 13 in die Vakuumpumpe 16 gesaugt und von dort zum Abluftauslass 5 transportiert. Die Sauerstoffversorgungsvorrichtung kann zusätzlich mit einem Sauerstoff-Pufferspeicher 21 ausgestattet sein, in dem überschüssiger Sauerstoff gespeichert wird. Vor dem Sauerstoffauslass 4 ist zur zusätzlichen Filterung der Luft eine HEPA-Filter 22 im Gasleitungssystem 6 angeordnet.

Die Erfindung ist nicht auf eine der vorbeschriebenen Ausführungsformen beschränkt, sondern in vielfältiger Weise abwandelbar.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

**Bezugszeichenliste**

| | |
|---|---|
| Sauerstoffversorgungsvorrichtung | 1 |
| Sauerstoffkonzentrator | 2 |
| Lufteinlass | 3 |
| Sauerstoff-Auslass | 4 |
| Abluftauslass | 5 |
| Gasleitungssystem | 6 |
| Gehäuse | 7 |
| Ansaugstutzen | 30 |
| Filtereinheit | 8 |
| Gewindeanschluss | 9 |
| Stromquelle | 10 |
| Erste Pumpe | 11 |
| Erkennungsmechanismus, Schalter | 12 |
| Erster Verteiler | 13 |
| Adsorbereinheit | 14 |
| Zweiter Verteiler | 15 |
| Zweite Pumpe | 16 |
| Zweiwegeventil | 17 |
| Schlauch zum Patienten | 20 |
| Sauerstoff-Pufferspeicher | 21 |
| HEPA-Filter | 22 |

## Patentansprüche

1. Tragbare Sauerstoffversorgungsvorrichtung (1) zur Versorgung eines Patienten umfassend
- einen Sauerstoffkonzentrator (2),
- mindestens einen Lufteinlass (3), durch den Umgebungsluft in die Sauerstoffversorgungsvorrichtung transportiert wird,
- mindestens einen Sauerstoff-Auslass (4), durch den angereicherter Sauerstoff aus der Sauerstoffversorgungsvorrichtung (1) transportiert wird,
- mindestens einen Abluftauslass (5), durch den Abluft aus der Sauerstoffversorgungsvorrichtung (1) transportiert wird,
- ein Gasleitungssystem (6), das mit dem mindestens einen Lufteinlass (3), dem Auslass (4) und dem Sauerstoffkonzentrator (2) fluidisch verbunden ist,
- eine Stromquelle (10),
- mindestens eine Pumpe (11, 16),
- ein Gehäuse (7), wobei alle Komponenten der Sauerstoffversorgungsvorrichtung (1) im oder am Gehäuse (7) angeordnet sind,
- wobei der Lufteinlass (3) einen Ansaugstutzen (30) aufweist, durch den die Umgebungsluft in die Sauerstoffversorgungsvorrichtung (1) gesogen wird,
**dadurch gekennzeichnet, dass** die Sauerstoffversorgungsvorrichtung (1) eine Filtereinheit (8) aufweist, die Filtereinheit (8) vor dem Lufteinlass (3) entweder direkt am Ansaugstutzen (30) oder mit einem Adapter am Ansaugstutzen (30) befestigt ist und die Filtereinheit (8) ausgewählt ist aus NBC-Filter, Kombinationsfilter und Partikelfilter.

2. Sauerstoffversorgungsvorrichtung (1) gemäß Patentanspruch 1, **dadurch gekennzeichnet, dass** die Sauerstoffversorgungsvorrichtung (1) ein mobiles Gerät ist, das als Stromquelle mindestens eine Batterieeinheit (10) oder einen Akkumulator (10) aufweist und ein stoßfestes Gehäuse (7) hat.

3. Sauerstoffversorgungsvorrichtung (1) gemäß Patentanspruch 1 oder 2, **dadurch gekennzeichnet, dass** die Filtereinheit (8) eine Filtereinheit für eine Atemschutzmaske/NBC-Schutzmaske ist, insbesondere eine NBC-Filtereinheit, eine Partikelfiltereinheit oder eine Kombinationsfiltereinheit ist.

4. Sauerstoffversorgungsvorrichtung (1) gemäß einem der vorhergehenden Patentansprüche, **dadurch gekennzeichnet, dass** der Ansaugstutzen (30) einen Gewindeanschluss (9) hat, der auf der der Umgebungsluft zugewandten Seite des Lufteinlasses (3) angeordnet ist.

5. Sauerstoffversorgungsvorrichtung (1) gemäß Patentanspruch 4, **dadurch gekennzeichnet, dass** der Gewindeanschluss (9) ein Gewindeanschluss Rd40 nach Norm EN 148 Teil 1) ist.

6. Sauerstoffversorgungsvorrichtung (1) gemäß einem der vorhergehenden Patentansprüche, **dadurch gekennzeichnet, dass** im Ansaugstutzen (30) ein Erkennungsmechanismus (11) angeordnet ist, wobei der Erkennungsmechanismus (11) erkennt, ob eine Filtereinheit (8) eingesetzt ist und ggf. zusätzlich erkennt welcher Filtertyp eingesetzt ist.

7. Sauerstoffversorgungsvorrichtung (1) gemäß Patentanspruch 6, **dadurch gekennzeichnet, dass** der Erkennungsmechanismus (11) ein mechanischer Druckschalter im Gewindeverlauf des Ansaugstutzens (30) ist.

8. Sauerstoffversorgungsvorrichtung (1) gemäß einem der Patentansprüche 6 oder 7, **dadurch gekennzeichnet, dass** der Erkennungsmechanismus (11) mit einer Steuereinheit verbunden ist und die Steuereinheit die Leistung des Sauerstoffkonzentrator (2) steuert und ggf. mit einer Informationseinheit verbunden ist, die eine Information zur Anwesenheit einer Filtereinheit und/oder der veränderten Leistung des Sauerstoffkonzentrators an den Anwender gibt.

9. Sauerstoffversorgungsvorrichtung (1) gemäß einem der vorhergehenden Patentansprüche, **dadurch gekennzeichnet, dass** der Sauerstoffkonzentrator (2) ein Druckwechseladsorptionssystem (PSA) oder ein Vakuum- Druckwechseladsorptionssystem (VPSA) ist und eine oder mehrere Adsorbersäulen aufweist.

10. Sauerstoffversorgungsvorrichtung (1) gemäß einem der vorhergehenden Patentansprüche, **dadurch gekennzeichnet, dass** die Sauerstoffversorgungsvorrichtung einen oder mehrere Sensoren im Gehäuse aufweist, die bevorzugt mit dem Gasleitungssystem verbunden sind.

11. Sauerstoffversorgungssystem aufweisen eine Sauerstoffversorgungsvorrichtung gemäß einem der Patentansprüche 1 bis 10 und eine fahrzeugtaugliche Gerätehalterung mit Sicherung.

## Geänderte Patentansprüche

### Geänderte Patentansprüche gemäss Regel 137(2) EPÜ.

1. Tragbare Sauerstoffversorgungsvorrichtung (1) zur Versorgung eines Patienten umfassend
- einen Sauerstoffkonzentrator (2),
- mindestens einen Lufteinlass (3), durch den Umgebungsluft in die Sauerstoffversorgungsvorrichtung transportiert wird,
- mindestens einen Sauerstoff-Auslass (4), durch den angereicherter Sauerstoff aus der Sauerstoffversorgungsvorrichtung (1) transportiert wird,
- mindestens einen Abluftauslass (5), durch den Abluft aus der Sauerstoffversorgungsvorrichtung (1) transportiert wird,
- ein Gasleitungssystem (6), das mit dem mindestens einen Lufteinlass (3), dem Auslass (4) und dem Sauerstoffkonzentrator (2) fluidisch verbunden ist,
- eine Stromquelle (10),
- mindestens eine Pumpe (11, 16),
- ein Gehäuse (7), wobei alle Komponenten der Sauerstoffversorgungsvorrichtung (1) im oder am Gehäuse (7) angeordnet sind,
- wobei der Lufteinlass (3) einen Ansaugstutzen (30) aufweist, durch den die Umgebungsluft in die Sauerstoffversorgungsvorrichtung (1) gesogen wird,
wobei die Sauerstoffversorgungsvorrichtung (1) eine Filtereinheit (8) aufweist, die Filtereinheit (8) vor dem Lufteinlass (3) entweder direkt am Ansaugstutzen (30) oder mit einem Adapter am Ansaugstutzen (30) befestigt ist
**dadurch gekennzeichnet, dass** die Filtereinheit (8) eine Filtereinheit für eine Atemschutzmaske ist und die Filtereinheit (8) ausgewählt ist aus NBC-Filter, Kombinationsfilter und Partikelfilter.

2. Sauerstoffversorgungsvorrichtung (1) gemäß Patentanspruch 1, **dadurch gekennzeichnet, dass** die Sauerstoffversorgungsvorrichtung (1) ein mobiles Gerät ist, das als Stromquelle mindestens eine Batterieeinheit (10) oder einen Akkumulator (10) aufweist und ein stoßfestes Gehäuse (7) hat.

3. Sauerstoffversorgungsvorrichtung (1) gemäß Patentanspruch 1 oder 2, **dadurch gekennzeichnet, dass** die Filtereinheit (8) eine NBC-Filtereinheit, eine Partikelfiltereinheit oder eine Kombinationsfiltereinheit ist.

4. Sauerstoffversorgungsvorrichtung (1) gemäß einem der vorhergehenden Patentansprüche, **dadurch gekennzeichnet, dass** der Ansaugstutzen (30) einen Gewindeanschluss (9) hat, der auf der der Umgebungsluft zugewandten Seite des Lufteinlasses (3) angeordnet ist.

5. Sauerstoffversorgungsvorrichtung (1) gemäß Patentanspruch 4, **dadurch gekennzeichnet, dass** der Gewindeanschluss (9) ein Gewindeanschluss Rd40 nach Norm EN 148 Teil 1) ist.

6. Sauerstoffversorgungsvorrichtung (1) gemäß einem der vorhergehenden Patentansprüche, **dadurch gekennzeichnet, dass** im Ansaugstutzen (30) ein Erkennungsmechanismus (11) angeordnet ist, wobei der Erkennungsmechanismus (11) erkennt, ob eine Filtereinheit (8) eingesetzt ist und ggf. zusätzlich erkennt welcher Filtertyp eingesetzt ist.

7. Sauerstoffversorgungsvorrichtung (1) gemäß Patentanspruch 6, **dadurch gekennzeichnet, dass** der Erkennungsmechanismus (11) ein mechanischer Druckschalter im Gewindeverlauf des Ansaugstutzens (30) ist.

8. Sauerstoffversorgungsvorrichtung (1) gemäß einem der Patentansprüche 6 oder 7,
**dadurch gekennzeichnet, dass** der Erkennungsmechanismus (11) mit einer Steuereinheit verbunden ist und die Steuereinheit die Leistung des Sauerstoffkonzentrator (2) steuert und ggf. mit einer Informationseinheit verbunden ist, die eine Information zur Anwesenheit einer Filtereinheit und/oder der veränderten Leistung des Sauerstoffkonzentrators an den Anwender gibt.

9. Sauerstoffversorgungsvorrichtung (1) gemäß einem der vorhergehenden Patentansprüche, **dadurch gekennzeichnet, dass** der Sauerstoffkonzentrator (2) ein Druckwechseladsorptionssystem (PSA) oder ein Vakuum- Druckwechseladsorptionssystem (VPSA) ist und eine oder mehrere Adsorbersäulen aufweist.

10. Sauerstoffversorgungsvorrichtung (1) gemäß einem der vorhergehenden Patentansprüche, **dadurch gekennzeichnet, dass** die Sauerstoffversorgungsvorrichtung einen oder mehrere Sensoren im Gehäuse aufweist, die bevorzugt mit dem Gasleitungssystem verbunden sind.

11. Sauerstoffversorgungssystem aufweisen eine Sauerstoffversorgungsvorrichtung gemäß einem der Patentansprüche 1 bis 10 und eine fahrzeugtaugliche Gerätehalterung mit Sicherung.
